(19) Europäisches Patentamt
European Patent Office
Office européen des brevets

(11) Veröffentlichungsnummer: **0 044 495**
**B1**

# (12) EUROPÄISCHE PATENTSCHRIFT

(45) Veröffentlichungstag der Patentschrift: **23.11.83**

(51) Int. Cl.³: **C 07 J 1/00**, A 61 K 31/565

(21) Anmeldenummer: **81105433.7**

(22) Anmeldetag: **11.07.81**

(54) **16-Beta-Ethylsteroide, diese enthaltende Präparate und Verfahren zu ihrer Herstellung.**

(30) Priorität: **21.07.80 DE 3027908**

(43) Veröffentlichungstag der Anmeldung: **27.01.82 Patentblatt 82/4**

(45) Bekanntmachung des Hinweises auf die Patenterteilung: **23.11.83 Patentblatt 83/47**

(84) Benannte Vertragsstaaten: **AT BE CH DE FR GB IT LI LU NL SE**

(56) Entgegenhaltungen:
**DE - B - 2 100 319**
**US - A - 3 086 027**

**JOURNAL OF THE AMERICAN CHEMICAL SOCIETY, Band 82, Nr. 9, 11. Mai 1960, Seiten 2402-2403 Washington D.C., U.S.A. MEL PERELMAN et al.: "A new class of active steroids: The 19-NOR-Delta 4,9-3-ketosteroids"**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Neef, Günter, Dr., Darmstädter Strasse 9, D-1000 Berlin 15 (DE)**
Erfinder: **Steinbeck, Hermann, Dr., Hammerstrasse 46, D-1000 Berlin 37 (DE)**

**EP 0 044 495 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 16-Beta-Ethylsteroide, diese enthaltende Präparate und Verfahren zu ihrer Herstellung.

Die Erfindung betrifft die in den Ansprüchen gekennzeichneten Gegenstände.

19-Nor-4,9(10)-3-Ketosteroide ohne 16β-Ethylgruppe sind beschrieben in J. of the American Chemical Society 82 (1960) 2402–2403 und in US-A 3 086 027 als Verbindungen mit anaboler, antiöstrogener und gestagener Aktivität.

Demgegenüber wurde gefunden, dass die erfindungsgemässen 16β-Ethylsteroide der allgemeinen Formel I starke antiandrogene Wirkung besitzen.

Unter einer Acylgruppe R in Formel I gemäss Anspruch 1 sollen die Reste von in der Steroidchemie gebräuchlichen Säuren verstanden werden. Beispielsweise genannt seien die Reste von organischen Carbon- und Sulfonsäuren mit 1 bis 17 Kohlenstoffatomen, vorzugsweise von organischen Carbonsäuren mit 1 bis 7 Kohlenstoffatomen, wie Formyl, Acetyl, Propionyl, Butyryl, Isobutyryl, Valeryl, Hexanoyl, Heptanoyl, Chloracetyl, Glykoloyl, Succinyl, Glutaryl, Adipoyl, Benzoyl usw.

Alkylgruppen in der Bedeutung von R sollen vorzugsweise 1 bis 5 Kohlenstoffatome enthalten und gegebenenfalls durch ein Sauerstoffatom unterbrochen sein. Beispiele für die gegebenenfalls durch ein Sauerstoffatom unterbrochene Alkylgruppe R sind Methyl, Ethyl, Methoxymethyl, Methoxyethyl, Ethoxyethyl, Propyl, Butyl, Pentyl usw.

Die neuen Verbindungen der allgemeinen Formel I besitzen starke antiandrogene Wirkung. Beispielsweise ist 16β-Ethyl-17β-hydroxy-4,9(10)-estradien-3-on stärker wirksam als das entsprechende in 9(10)-Stellung gesättigte Steroid. Zur Bestimmung der antiandrogenen Wirkung wurde die Hemmung der durch Testosteronpropionat bedingten Zunahme des Samenblasen- und Prostatagewichts von Ratten nach subkutaner Applikation bestimmt. Wie die folgende Tabelle zeigt, ist das erfindungsgemässe 16β-Ethyl-17β-hydroxy-4,9(10)-estradien-3-on (A) mit 0,3 mg pro Tier und Tag signifikant wirksamer als das aus der deutschen Auslegeschrift 2 100 319 bekannte 16β-Ethyl-17β-hydroxy-4-estren-3-on (B).

| Verbindung | Dosis (mg/Tag) | Testosteronpropionat-Hemmung | |
|---|---|---|---|
| | | Samenblase | Prostata |
| A | 0,3 | 34% | 18% |
| B | 0,3 | 13% | 0% |

Auf Grund der günstigen antiandrogenen Eigenschaften sind die erfindungsgemässen Verbindungen gut geeignet zur Behandlung von Krankheiten, die durch Androgene bedingt oder von Androgenen abhängig sind. Beispielsweise können die Verbindungen der allgemeinen Formel I zur Behandlung der altersbedingten Prostatahyperplasie in Mengen von etwa 10 bis 200 mg/Tag verwendet werden. Ferner kommen die Behandlung von Akne, Hirsutismus und Alopecie infrage.

Die Wirkstoffe können mit den in der galenischen Pharmazie üblichen Zusätzen, Trägersubstanzen und/oder Geschmackskorrigentien nach an sich bekannten Methoden zu den üblichen Applikationsformen verarbeitet werden.

Für die bevorzugte orale Applikation kommen insbesondere Tabletten, Dragées, Kapseln, Pillen, Suspensionen oder Lösungen infrage.

Für die parenterale, insbesondere intramuskulare Applikation sind ölige Lösungen, wie zum Beispiel Sesamöl- oder Rizinusöllösungen, geeignet. Zur Erhöhung der Löslichkeit können Lösungsvermittler, wie zum Beispiel Benzylbenzoat oder Benzylalkohol, zugesetzt werden. Von den Verbindungen der allgemeinen Formel I sind die 17β-Acylderivate (R = Acyl) in Ölen besonders leicht löslich. Sie besitzen in öliger Lösung nach intramuskularer Applikation eine protrahierte Wirkung. Die Konzentration der Wirkstoffe in öliger Lösung beträgt etwa 10–200 mg/ml.

Das Verfahren zur Herstellung von 16β-Ethylsteroiden der allgemeinen Formel I wird in an sich bekannter Weise durchgeführt und ist dadurch gekennzeichnet, dass man eine 16β-Ethylverbindung der Formel II

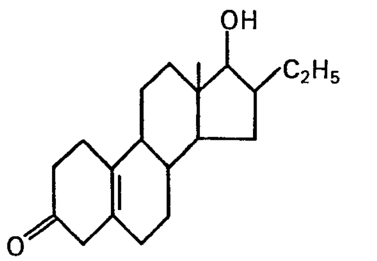

(II),

bromiert und dehydrobromiert und gegebenenfalls die 17-Hydroxygruppe verestert oder verethert.

Die Bromierung und Dehydrobromierung der Verbindung gemäss Formel II wird vorzugsweise mit Pyridinhydrobromid-perbromid in Gegenwart einer organischen Base durchgeführt. Als organische Base kommt beispielsweise Pyridin, Collidin oder Triäthylamin in Betracht. Vorzugsweise verwendet man Pyridin und arbeitet bei etwa 0 bis 100 °C. Die Einführung der zusätzlichen Doppelbindung erfolgt unter Ausbildung der $\Delta^{4,9(10)}$-Gruppierung.

Die Veresterung der freien 17β-Hydroxygruppe in der Verbindung gemäss Formel I kann durch Behandlung mit einem reaktiven Säurederivat, zum Beispiel einem Säureanhydrid oder -halogenid, in Gegenwart einer Base wie Pyridin, 4-Dimethylaminopyridin oder Collidin bei Temperaturen von etwa 20 bis 120 °C erfolgen.

Zur Veretherung der 17β-Hydroxygruppe dienen alkylierende Verbindungen, wie zum Beispiel Alkylhalogenide. Die Veretherung erfolgt in an

sich bekannter Weise in Gegenwart einer starken Base, wie Natronlauge, unter Verwendung eines polaren Lösungsmittels, wie Hexamethylphosphorsäuretriamid, bei 0–50 °C oder in Gegenwart einer starken Base, wie Natriumhydrid, unter Verwendung eines Ethers, wie Tetrahydrofuran, bei 30–100 °C.

Zur Herstellung von Alkylethern, deren Kohlenstoffkette durch ein Sauerstoffatom unterbrochen und gegebenenfalls ringgeschlossen ist, wird die Hydroxyverbindung mit Dihydropyran oder Alkylvinylethern in Gegenwart einer starken Säure, wie p-Toluolsulfonsäure oder Phosphoroxychlorid, in die entsprechenden Tetrahydropyranyl- oder Alkoxyethylether überführt. Die Reaktion wird vorzugsweise in Gegenwart von inerten Lösungsmitteln, wie Chloroform, Dichlormethan, Tetrahydrofuran, Dioxan etc., bei einer Reaktionstemperatur von −20 °C bis 100 °C durchgeführt. Zur Herstellung von Methoxymethylethern wird die Hydroxyverbindung zum Beispiel mit Formaldehyddimethylacetal in wasserfreiem Dichlormethan in Gegenwart von Phosphorpentoxid bei Raumtemperatur umgesetzt.

Das als Ausgangsmaterial des erfindungsgemässen Verfahrens dienende 16β-Ethyl-17β-hydroxy -5(10)-estren-3-on der Formel II wird nach an sich bekannten Methoden aus 16β-Ethyl-3 methoxy 1,3,5(10) estratrien 17β ol (16β-Ethyl-östradiol-3-methylether – deutsche Auslegeschrift 2 856 578 –) beispielsweise durch Birch-Reduktion und saure Hydrolyse des Birch-Produkts wie folgt hergestellt:

a) Eine Lösung von 10,0 g 16β-Ethyl-3-methoxy-1,3,5(10)-estratrien-17β-ol in 200 ml Tetrahydrofuran und 20 ml tert.-Butanol wird bei −45 °C zu 500 ml Ammoniak getropft. Anschliessend gibt man portionsweise 6,4 g Lithium hinzu und rührt danach 6 Stunden bei −40 °C. Zur Aufarbeitung lässt man Ammoniak über Nacht verdampfen, nimmt den Rückstand in ca. 1,5 l Wasser auf und extrahiert mit Essigester. Die Essigester-Extrakte werden mit gesättigter Kochsalzlösung gewaschen, über Natriumsulfat getrocknet und eingeengt. Man erhält 9,8 g 16β-Ethyl-3-methoxy-2,5(10)-estradien-17β-ol als farbloses Öl, das ohne weitere Reinigung in die Folgestufe eingesetzt wird.

b) Eine Lösung von 9,8 g des obigen Rohprodukts in 400 ml Aceton wird nach Zusatz einer Lösung von 7,6 g Oxalsäure in 100 ml Wasser 60 Minuten bei 40 °C gerührt. Anschliessend engt man im Vakuum auf ein Volumen von ca. 200 ml ein, giesst in Wasser und extrahiert mit Essigester. Der nach dem Einengen verbleibende Rückstand besteht aus 9,1 g 16β-Ethyl-17β-hydroxy-5(10)-estren-3-on.

Beispiel 1

9,1 g 16β-Ethyl- 17β-hydroxy- 5(10)-estren-3-on werden in 480 ml Pyridin gelöst und unter Eiswasserkühlung portionsweise mit 14,4 g Pyridinhydrobromid-perbromid versetzt. Nach Zugabe rührt man 2 Stunden bei 50 °C unter Argon, giesst anschliessend in ca. 3 l Wasser und extrahiert mit Essigester. Die Essigester-Extrakte werden mit 2n-HCl und Kochsalzlösung gewaschen, über Natriumsulfat getrocknet und eingeengt. Nach Chromatographie des Rückstandes an Kieselgel mit Benzin/Essigester 0–15% erhält man 7,2 g 16β-Ethyl-17β-hydroxy-4,9(10)-estradien-3-on vom Schmelzpunkt 109–110 °C.

Beispiel 2

Eine Lösung von 2,5 g 16β-Ethyl-17β-hydroxy-4,9(10)-estradien-3-on in 10 ml Acetanhydrid und 3 ml Pyridin wird 3 Stunden bei 60 °C gerührt. Danach tropft man die Reaktionslösung vorsichtig in ca. 100 ml gesättigter $NaHCO_3$-Lösung und extrahiert mit Essigester. Nach Kristallisation des Rückstandes aus Essigester/Diisopropylether erhält man 2,4 g 17β-Acetoxy-16β-ethyl-4,9(10)-estradien-3-on vom Schmelzpunkt 105–106 °C.

Beispiel 3

Eine Suspension von 15 g 16β-Ethyl-17β-hydroxy-4,9(10)-estradien-3-on in 450 ml Methylenchlorid, 100 ml Methylal und 30 g Celite wird unter Eiskühlung portionsweise mit 15 g Phosphorpentoxid versetzt. Anschliessend rührt man 60 Minuten bei 0–5 °C, filtriert, versetzt das Filtrat mit 15 ml Triethylamin, wäscht es mehrfach mit gesättigter Kochsalzlösung, trocknet über Natriumsulfat und engt ein. Nach Kristallisation des Rohprodukts aus Essigester/Diisopropylether erhält man 14,2 g 16β-Ethyl-17β-methoxymethoxy-4,9(10)-estradien-3-on vom Schmelzpunkt 101–103 °C.

Beispiel 4

Eine Lösung von 2 g 16β-Ethyl-17β-hydroxy-4,9(10)-estradien-3-on in 30 ml Toluol wird nach Zusatz von 5 g Capronsäureanhydrid und 2,5 g 4-Dimethylaminopyridin 3 Stunden bei 60 °C gerührt. Anschliessend giesst man in gesättigte $NaHCO_3$-Lösung und extrahiert mit Essigester. Nach Chromatographie des Rohprodukts an Kieselgel mit Hexan/Essigester 0–20% erhält man 1,8 g 16β-Ethyl-17β-hexanoyloxy-4,9(10)-estradien-3-on vom Schmelzpunkt 74–76 °C (aus Hexan).

**Patentansprüche für die Vertragsstaaten: BE, CH, GB, IT, LI, LU, NL, SE**

1. 16β-Ethylsteroide der allgemeinen Formel I worin

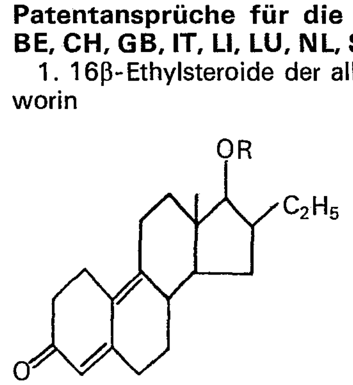

(I),

R ein Wasserstoffatom, eine Acyl- oder eine gegebenenfalls durch ein Sauerstoffatom unterbrochene Alkylgruppe oder die Tetrahydropyranylgruppe bedeuten.

2. 16β-Ethyl-17β-hydroxy-4,9(10)-estradien-3-on.

3. 17β-Acetoxy-16β-ethyl-4,9(10)-estradien-3-on.

4. 16β-Ethyl-17β-hexanoyloxy-4,9(10)-estradien-3-on.

5. 16β-Ethyl-17β-methoxymethoxy-4,9(10)-estradien-3-on.

6. Pharmazeutische Präparate auf Basis von Verbindungen gemäss Anspruch 1–5.

7. Verfahren zur Herstellung von 16β-Ethylsteroiden der allgemeinen Formel I, dadurch gekennzeichnet, dass man eine 16β-Ethylverbindung der Formel II

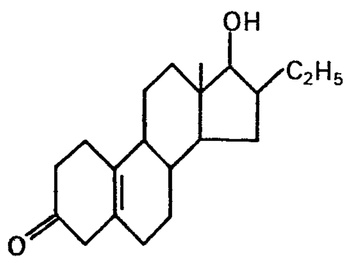

(II),

bromiert und dehydrobromiert und gegebenenfalls die 17-Hydroxygruppe verestert oder verethert.

**Patentanspruch für den Vertragsstaat: AT**

Verfahren zur Herstellung von 16β-Ethylsteroiden der allgemeinen Formel I

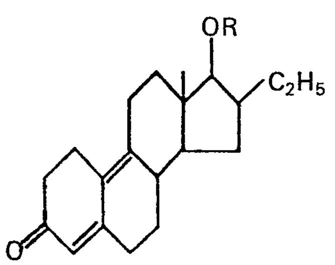

(I),

worin

R ein Wasserstoffatom, eine Acyl- oder eine gegebenenfalls durch ein Sauerstoffatom unterbrochene Alkylgruppe oder die Tetrahydropyranylgruppe bedeuten, dadurch gekennzeichnet, dass man eine 16β-Ethylverbindung der Formel II

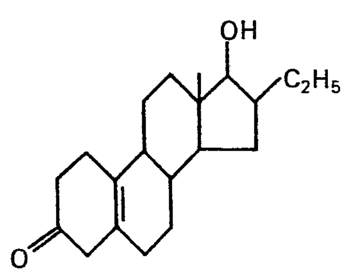

(II),

bromiert und dehydrobromiert und gegebenenfalls die 17-Hydroxygruppe verestert oder verethert.

**Revendications pour les Etats contractants BE, CH, DE, FR, GB, IT, LI, LU, NL, SE**

1. Ethyl-16β stéroïdes répondant à la formule générale I

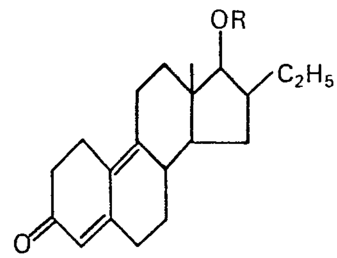

(I),

dans laquelle R représente un atome d'hydrogène, un radical acyle, un radical alkyle éventuellement interrompu par un atome d'oxygène, ou un radical tétrahydropyrannyle.

2. Ethyl-16β hydroxy-17β œstradiène-4,9(10) one-3.

3. Ethyl-16β acétoxy-17β œstradiène-4,9(10) one-3.

4. Ethyl-16β hexanoyloxy-17β œstradiène-4,9(10) one-3.

5. Ethyl-16β méthoxyméthoxy-17β œstradiène-4,9(10) one-3.

6. Médicaments caractérisés en ce qu'ils contiennent un composé selon l'une quelconque des revendications 1 à 5.

7. Procédé de préparation d'éthyl-16β stéroïdes de formule générale I, procédé caractérisé en ce qu'on brome et déshydrobrome un composé éthylé en 16β qui répond à la formule II

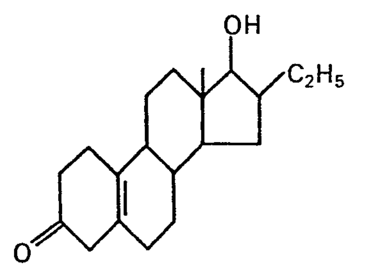

(II),

et on estérifie ou éthérifie éventuellement le groupe hydroxy en 17.

**Revendication pour l'Etat contractant AT**

Procédé de préparation d'éthyl-16β stéroïdes répondant à la formule générale I

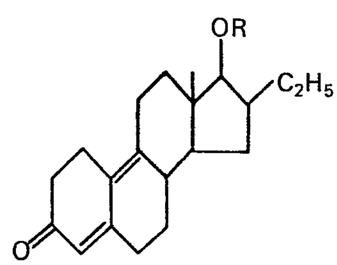

(I),

dans laquelle R représente un atome d'hydrogène, un radical acyle, un radical alkyle éventuellement interrompu par un atome d'oxygène, ou un radical

tétrahydropyrannyle, procédé caractérisé en ce qu'on brome et déshydrobrome un composé éthylé en 16β qui répond à la formule II

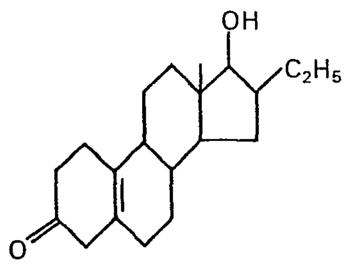

(II),

et on estérifie ou éthérifie éventuellement le groupe hydroxy en 17.

**Claims for the Contracting States BE, CH, DE, FR, GB, IT, LI, LU, NL, SE**

1. 16β-Ethyl steroids of the general formula I

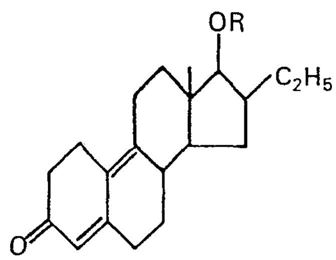

(I),

in which

R denotes a hydrogen atom, an acyl group, an alkyl group optionally interrupted by an oxygen atom or a tetrahydropyranyl group.

2. 16β-Ethyl-17β-hydroxy-4,9(10)-oestra-dien-3-one.

3. 17β-Acetoxy-16β-ethyl-4,9(10)-oestra-dien-3-one.

4. 16β-Ethyl-17β-hexanoyloxy-4,9(10)oestra-dien-3-one.

5. 16β-Ethyl-17β-methoxymethoxy-4,9(10)-oestradien-3-one.

6. Pharmaceutical preparations containing a compound according to claims 1 to 5.

7. Process for the manufacture of 16β-ethyl steroids of the general formula I, characterised in that a 16β-ethyl compound of formula II

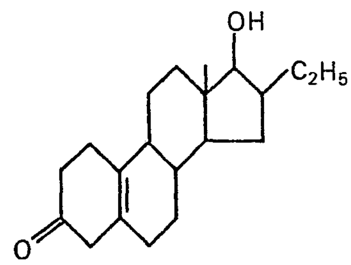

(II),

is brominated and dehydrobrominated and optionally the 17-hydroxy group is esterified or etherified.

**Claim for the Contracting State AT**

Process for the manufacture of 16β-ethyl steroids of the general formula I

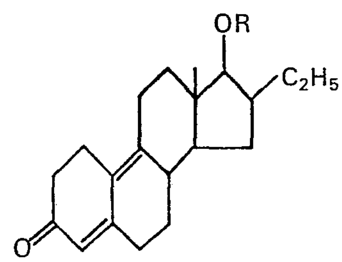

(I),

in which

R denotes a hydrogen atom, an acyl group, an alkyl group optionally interrupted by an oxygen atom or a tetrahydropyranyl group, characterised in that a 16β-ethyl compound of formula II

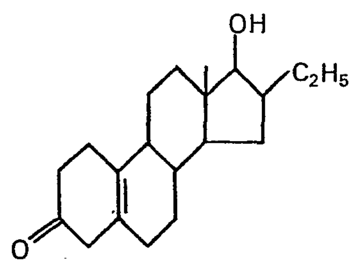

(II),

is brominated and dehydrobrominated and optionally the 17-hydroxy group is esterified or etherified.